Europäisches Patentamt

European Patent Office

Office européen des brevets

(11) Publication number: **0 419 234 A2**

# EUROPEAN PATENT APPLICATION

(21) Application number: **90310255.6**

(22) Date of filing: **19.09.90**

(51) Int. Cl.5: **C12M 3/00**

(30) Priority: **22.09.89 GB 8921458**

(43) Date of publication of application:
**27.03.91 Bulletin 91/13**

(84) Designated Contracting States:
**AT BE CH DE DK ES FR GB GR IT LI LU NL SE**

(71) Applicant: **THE UNIVERSITY OF STRATHCLYDE**
**50 George Street**
**Glasgow G1 1BA, Scotland(GB)**

(72) Inventor: **Cousins, Roderick Bruce**
**47 Westbourne Gardens**
**Glasgow, G12 9XQ(GB)**

(74) Representative: **Horner, Martin Grenville et al**
**Cruikshank & Fairweather 19 Royal**
**Exchange Square**
**Glasgow G1 3AE Scotland(GB)**

(54) Cell culture apparatus.

(57) A cell culture apparatus comprises a chamber (1) for containing a cell culture having a first bundle of hollow semipermeable fibres (5) passing through the chamber for aqueous dialysing liquid for removing waste products (and optionally supplying nutrients), and a second bundle of hollow semipermeable fibres (7) for carrying an oxygen-containing gas and removing gaseous waste products from the cell culture. A third bundle (16) may also be included. The chamber may be cylindrical, or may comprise layers of fibres at right angles, or may be formed of a stack of plates each having a series of fibres therein.

## CELL CULTURE APPARATUS

The present invention relates to a cell culture apparatus for growing cells in vitro with the use of hollow semipermeable fibres.

U.S. patent specifications 3821087 and 3883393 disclose the culturing of cells in a culture chamber. A bundle of semipermeable hollow fibres passes through the chamber, through which nutrient solution containing oxygen is flowed. Oxygen and nutrients diffuse from the flowing oxygenated nutrient medium through the semipermeable cell walls into the chamber, whilst cell waste products e.g. lactic acid, and desirable products e.g. hormones, diffuse from the chamber through the fibre walls into the flowing solution. The object of these prior art devices is to grow three-dimensional layers of cells attached to the semipermeable fibres in an attempt to produce organ-like structures.

In the known hollow fibre culture system it is conventional to use a culture medium containing a bicarbonate buffer to assist in the maintenance of the culture pH. The capacity of this buffer system is limited and this gives rise in practice to pH gradients within the essentially static culture space. In addition the nutrient stream in these prior art devices is also the carrier for the oxygen, which is required for culture growth. As it is sparingly soluble in aqueous solutions the culture can become depleted in oxygen towards the outlet end of the fibre bundle. Also this nutrient stream acts as the carrier for metabolic waste products; removing products such as lactate by diffusion across the membrane from the culture. Maintaining the levels of such metabolites within acceptable limits is achieved by continuously removing a proportion of the nutrient medium. This leads to poor utilisation of this expensive component.

It is an object of the present invention to provide a cell culture apparatus of the same general type but which mitigates these problems and allows more flexible control of the culture conditions.

The present invention provides a cell culture apparatus which comprises;
A cell culture apparatus which comprises;
- a chamber within which cells may be cultured in a lqiuid culture medium, the chamber having access port means for introducing nutrient and cell inoculum and for withdrawing cultured cells and cell-products;
- a first bundle of hollow semipermeable fibres contained within the chamber and having an inlet and an outlet for flowing a stream of aqueous liquid therethrough for removal of cell culture products, in use the products passing through the fibre walls from the cell culture medium into the flowing liquid stream within the fibres; and

- a second bundle of hollow semipermeable fibres within the chamber and having an inlet for an oxygenating gas stream, in use oxygen-containing gas passing through the hollow fibre walls from the gas stream into the culture medium and gaseous waste products passing from the culture medium into the gas stream.

The term "bundle" is to be interpreted broadly as including arrays of fibres in any suitable configuration.

The aqueous liquid as well as acting as a dialysing stream may as required also include nutrient solution.

In one embodiment of the invention the chamber is substantially cylindrical and the first and second bundles of fibres arranged to pass through the chamber in a parallel array with one fibre bundle placed above the other. Usually it is preferred to place the gas transfer fibres in the lower bundle. Alternatively the fibres of the first and second bundles can be intimately intermixed so that as far as possible, oxygen and nutrient are uniformly supplied to the nutrient medium around the fibres and concentration gradients are minimised.

In a second embodiment, the culture chamber is substantially rectangular and consists of a series of plates separated by suitably formed gaskets, which are compressed together between a top and a bottom plate secured and connected by means of tie rods. Strung across the open central area of each plate is a parallel layer of either the first or second type of fibre secured at each end by means of a suitable polymeric elastomer potting compound. The lumen of the fibres are connected to the inlet and outlet by means of manifold channels formed in the plate. These channels interconnect between the plates to form separate inlets and outlets for the first and second fibre types. For preference the plates are arranged alternately and the number of plates determine the volume of the reactor. Alternatively each plate may be so constructed to contain the two types of fibre arranged in two parallel layers with separate (possibly staggered) manifold channels formed in the upper and lower surface of each plate. Thus each plate has separate inlets and outlets for the two fibre types and these inlets and outlets interconnect with the matching inlets and outlets of every other plate and can be arranged so as to form a collection of fibres in parallel or in series.

In a third embodiment, the first and second bundles of fibres are arranged to run in substantially perpendicular directions. Usually, the fibres of each type are arranged as alternate single layers and are bonded to form a single unit with a common inlet and outlet for each fibre type. This arrangement increases the structural integrity of the fibres, which become a substantially self-supporting matrix. The fibres may also be arranged to run at any other angle from 0 - 90% to one another. Where there are more than two fibre bundles, these will usually be equiangularly spaced.

In the invention so far described, the majority of the essential nutrients can be supplied either indirectly via the first fibre bundle or alternatively they may be supplied directly to the culture chamber. In the second case there is a net outflow of liquid through the first bundle of semipermeable fibres to account for liquid introduced into the chamber as part of the nutrient solution. This net flow of liquid could be a disadvantage with certain fibre structures in that cells tend to be sucked into the pores of the microporous semipermeable fibres causing blockages or decreased throughput. It can therefore be advantageous to introduce nutrients into the culture medium by pure diffusion transport by providing a third bundle of hollow semipermeable fibres in the chamber through which the nutrient solution passes. Nutrients then diffuse into the culture medium through the fibre walls. In this way, there is no net bulk flow of liquid through the fibre walls. Alternatively by the correct choice of fibre type and pore size the third fibre bundle may be used to supply the complete nutrient requirements of the cell line under culture while at the same time removing higher molecular weight metabolites and products from the culture in a similar manner to that of the first bundle.

The fibres of the first bundle are generally formed of a hydrophilic semipermeable membrane material having an asymmetric membrane structure with the principal membrane surface on the inside of the fibre and a spongy outer layer. Typically, the fibre is formed from a polysulphone suitably treated to form a hydrophilic membrane. A similar material of the same or different pore size and molecular weight cut-off might be used for the fibres of the optional third bundle. The fibres of the second bundle for containing the oxygenating gas stream are preferably formed of a hydrophobic microporous material, particularly a polypropylene.

In the first preferred embodiment the length of the fibres is typically in excess of 100mm with an upper limit in the region of 500mm. The bundles would typically contain 100 - 1000 fibres and have dimensions of 280 microns diameter with a wall thickness of 40 microns. With a total culture volume of approximately 250 ml this gives a fibre surface area to culture volume ratio of 0.5-5.0 $cm^2/ml$ which contrasts with the prior art devices which typically have a surface area to volume ratio of approximately 50 $cm^2/ml$.

In the second preferred embodiment the fibres are affixed to plates and may be arranged in alternate layers of hydrophilic and hydrophobic fibres; and are of similar length to those of the first embodiment and have a similar surface to culture volume ratio. This arrangement allows individual plates to be tested for integrity, and facilitates altering the ratio of numbers of fibres in the bundles. For larger sizes a support mesh may be included.

In the third preferred embodiment the preferred arrangement of the fibres is a matrix of alternating layers of hydrophilic and hydrophobic fibres with the bulk movement of the culture at an angle perpendicular to this matrix. The device has a fibre surface area to culture volume ratio typically similar to that of the first device but the alternative mixing regime permits the device to be used with shorter fibre lengths, e.g. 50-250 mm.

In order to control the pH, it is conventional to include carbon dioxide in the oxygen-containing gas stream. The apparatus may also include appropriate pH and temperature sensors, as well as a port for seeding the culture medium with cells and for removing samples.

The apparatus may be used to grow cells themselves or to culture cells for their extracellular or intra-cellular products, such as enzymes, hormones, monoclonal antibodies etc. The cell products include undesired products of low molecular weight which pass into the flowing liquid stream. These waste products are removed by dialysis by the aqueous liquid stream, typically water or an isotonic salt solution, passing through the lumen of the hydrophilic fibre bundle. If desired, the aqueous liquid may include a basal nutrient medium. Desirable cell products may be arranged to be retained within the culture medium or diffuse into the flowing liquid stream by appropriate choice of pore size of the semipermeable fibre.

Undesirable gaseous waste products, such as carbon dioxide and ammonia, are removed in the second bundle of fibres.

In order to provide homogenous conditions within the culture chamber and to improve the mass transfer characteristics of the devices, the apparatus preferably includes a mixing means for moving the culture medium relative to the fibre bundles so that the transfer of gaseous and liquid nutrients and products is improved. In the first two embodiments, the chamber is only partially full of culture medium and is provided with a tilting or rocking mechanism to move the culture medium from side-to-side or end-to-end of the chamber (for example, at a frequency of 8-30 per minute). The space above the culture permits the culture to be moved freely by the gentle rocking action thus providing mixing without damage to the cells. As the space above the medium is sealed from the external gas supply, its volume is fixed by the balance between the internal pressure and the mean hydrostatic pressure of the nutrient line. The method of mixing in the first two embodiments provides the low shear regime necessary for the successful culture of mammalian cells and avoids the need for internal stirrers together with the associated seals. Alternatively, a vibration mechanism may be provided.

In a further preferred embodiment, the chamber is provided with deformable cell walls on opposite sides of the fibres and the cell walls are arranged to move inwards and outwards in unison so as to force the liquid culture medium passed the stationary fibre bundles. The cell walls may be deformed in unison by attaching to a suitable reciprocating hydraulic mechanism working at, for example, 0.25 to 1 Hz.

An important advantage of the present invention is that the gas and nutrient streams are separate, in contrast to rhe prior art. This allows the composition of the gas and nutrient streams to be controlled independently, thereby facilitating control of the composition of the culture medium. The introduction of carbon dioxide uniformly into the culture medium reduces the possibility of undesirable pH gradients within the culture medium. Moreover, as the mixing means provides near homogenous conditions within the chamber and all the principal streams, have been separated, it is possible to manipulate them individually, thus permitting concentrated nutrient feed streams to be used. Prior art constructions using a single fibre bundle require a separate device for introducing oxygen and carbon dioxide into the recirculating nutrient solution. This need is removed in the present invention which may be embodied in a unitary construction.

The culture apparatus is suitable for massproduction to provide a disposable item, which avoids the need for cleaning and resterilisation.

Embodiments of the present invention will now be described by way of example only with reference to the accompanying drawings wherein;

Figure 1 is a schematic view of a first embodiment having parallel fibre bundles, together with associated peripheral equipment;
Figure 2 is a longitudinal levation of a second embodiment having three parallel fibre bundles;
Figure 3 is a plan view;
Figure 4 is an end elevation;
Figure 5 is a perspective view of a third embodiment having two fibre bundles at right angles;
Figures 6 and 7 are respectively a longitudinal part-section and a cross-section of showing in detail an end of a tubular chamber having two different types of fibre bundle;
Figures 8 and 9 are analogous detailed views of a chamber having three different fibres bundles;
Figures 10 and 11 show respectively a series of fibre bundles mounted in a first type of frame and a cross-section of a corresponding culture apparatus according to a fourth embodiment;
Figures 12 and 13 show respectively a plan view and part cross-section on A-A of a second type of frame which includes two types of fibre bundle;
Figure 14 shows a third type of frame which also includes two types of fibre bundle, and Figures 15 and 16 are respectively sections on C-C and B-B;
Figure 17 is a schematic view of a fifth embodiment of the invention wherein nutrient medium is fed hydrostatically into one of the fibre bundles; and
Figure 18 is a schematic view of a six embodiment as employed in Example 1.

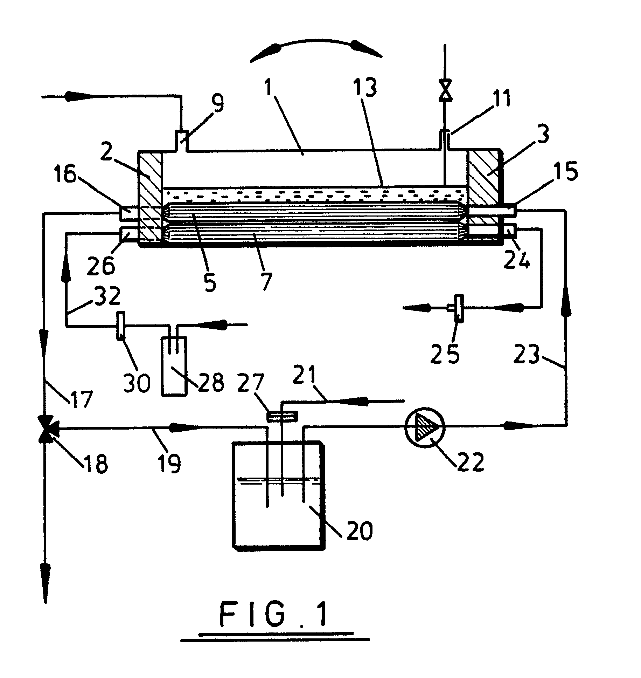

The cell culture apparatus shown schematically in Figure 1 comprises a rigid tubular or parallelsided chamber 1 constructed from a suitable biologically compatible material and sealed at each end by a respective end plate 2,3. The lower part of the chamber contains two loosely packed bundles 5, 7 of hollow semipermeable fibres extending along the full length of the chamber and passing through the end plates. The two bundles are arranged one above the other and consist of an upper hydrophilic fibre bundle 5 for dialysing liquid (and optionally nutrient) and a lower hydrophobic bundle 7 for oxygenating gas. The fibres are spaced such that the cells and medium can freely circulate around the fibres under the gentle mixing conditions caused by the rocking motion of the chamber, created by means of a rocking table (not shown) beneath the chamber. The bundles of hollow fibres are sealed to the chamber end plates by means of a suitable polymeric elastomer potting compound, such as a medical grade polyurethane which encapsulates the fibres at the point where they pass through the end plates in such a way as to form a gas and liquid seal. This also permits connection of the hollow fibres to the external circuit so that liquid and gas can be pumped through the fibre bundles.

The chamber is provided with an inlet 9 for nutrient solution and a sampling port 11 through which a syringe or other sampling device may be inserted for introducing cells or for sampling the culture medium. The chamber is only half filled with culture medium so as to enable mixing to occur under the action of the rocking means.

lhe first bundle of fibres 5 has a typical length of 240 mm (approximately 300-800 fibres) has an inlet 15 for saline (and optionally nutrient solution) and an outlet 16 for solution containing cell products, which passes through line 17 to bleed valve 18 where a portion is bled off and the remainder recycled through line 19 to dialysate reservoir 20. A make-up dialysate stream 21 is passed through a filter 27 before being introduced into the reservoir to compensate for the portion bled off. Dialysate is recirculated via pump 22 and line 23 to inlet 15 again.

The second bundle of hollow semipermeable fibres 7 (approximately 100 fibres) for supplying oxygen and $CO_2$ has an inlet 26 and outlet 24 protected by a bacterial filter 25. The air/oxygen stream from a suitable pressure source is mixed with a pressurised carbon dioxide stream controlled by means of a solenoid valve activated by a pH controller (not shown) dependent on the pH of the culture medium. This mixed stream is passed through a filter 30 before being passed through the fibre lumen via line 32 and inlet 26. Gas from the outlet 24 is vented to atmosphere preferably via a sanitising bubble chamber (not shown). To reduce vapour loss from the culture chamber the gas can if required be passed through a humidifying chamber 28 before entering the fibre lumen. Gas from the outlet 24 is vented to atmosphere.

Two distinct types of hollow fibre are employed in the construction of the apparatus. The first type is used for the dialysate (and optional nutrient) stream passing through first bundle 5 and comprises a thin walled hydrophilic semipermeable fibre having an assymetric membrane structure and a spongy outer layer. Typically, a fibre similar to those employed in high flux dialysers (such as a polysulphone fibre) will be used. These fibres have a wall thickness of about 40 microns, an internal lumen diameter of 180 microns and permit the transfer of solutes by both diffusion and convection. The average pore size of the membrane is selected so that the desired extra-cellular product during culture is retained within the culture chamber, while unwanted low molecular weight extracellular metabolites are able to pass through the semipermeable fibre wall into the dialysate stream flowing in the fibre lumen. The wall thickness, porosity and surface area of the fibre bundle is such that interfering or inhibitory metabolites can be maintained within manageable limits.

The second type of fibre is used in the second fibre bundle 7 for supplying oxygen and $CO_2$, and is made up of hydrophobic microporous hollow fibres. These fibres are typically constructed from a microporous polypropylene material and have dimensions similar to those of the hydrophilic fibres. The hydrophobic fibres are used to supply gaseous nutrients to the culture medium and to remove undesirable dissolved gaseous metabolites. In operation, a mixture of air and carbon dioxide is passed through the fibre lumen. The carbon dioxide is added to the air to maintain the pH of the culture medium and can be varied by means of a suitable pH controller monitoring directly the culture pH, so that culture conditions can be sustained within defined limits.

A reservoir (not shown) can be provided for the fresh nutrient, which is pumped from the reservoir either continuously or intermittently directly into the cell culture medium via inlet 9. This line can additionally be used to remove a proportion of the culture from time to time if desired. The liquid level within the chamber increases until the internal hydrostatic pressure is sufficient to maintain an equal outflow of liquid through the first bundle of fibres 5.

The complete reactor shown in Figure 1 is manufactured from materials which can be sterilised by steam in an autoclave or alternatively by circulating steam through the circuit under pressure.

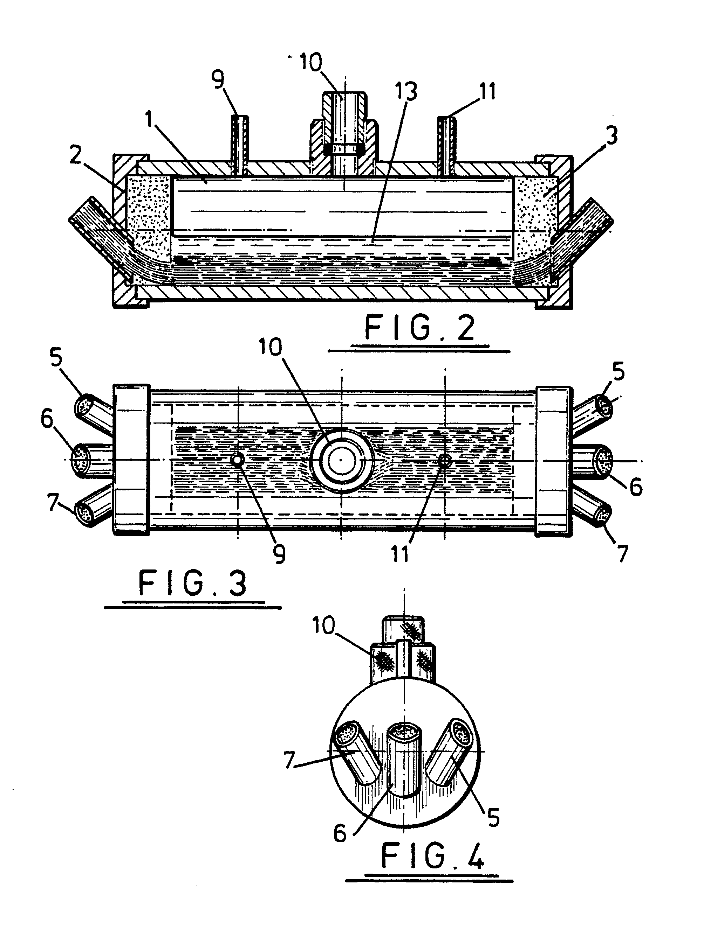

Figures 2 to 4 show a second embodiment of the same general type as the first embodiment shown in Figure 1, with the addition of a third bundle of fibres to handle a recirculating nutrient stream. Analogous parts are shown with the same reference number.

First and second bundles 5, 7 respectively handle the dialysate stream and gas stream. A third bundle 6 of hollow semipermeable fibres is attached to a nutrient solution reservoir (not shown) and recirculating pump for recirculating nutrient solution through the third bundle of fibres, whereby nutrients diffuse through the semipermeable membrane into the culture medium 13. The fibres of the three bundles are intimately intermixed so as to minimise concentration gradients of nutrient species within the culture medium.

A port 10 is provided for a pH sensor or thermometer.

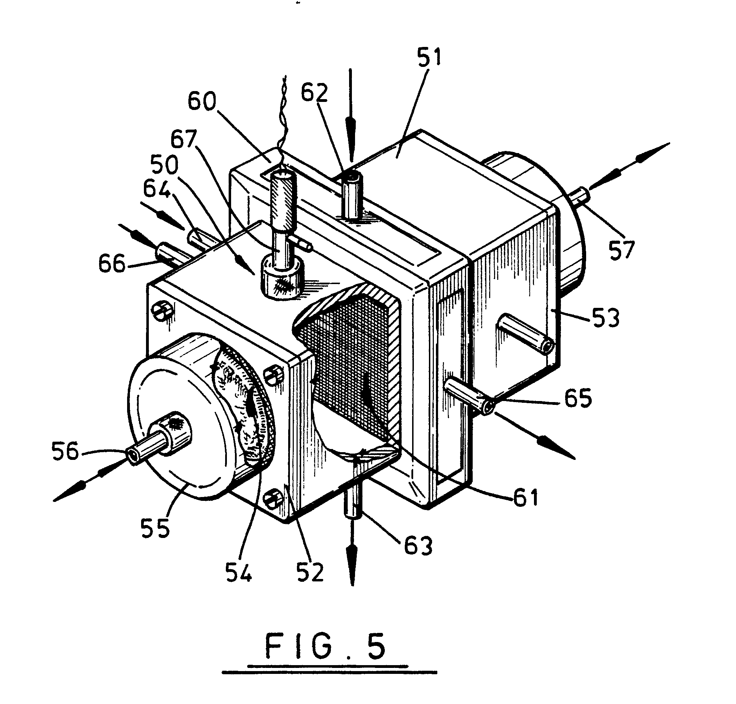

Figure 5 shows a third embodiment comprising a matrix of two fibre bundles arranged at right angles.

The culture chamber is formed of two halves 50 and 51, each being provided with a respective end plate 52, 53. Each end plate has a circular aperture sealed by means of a rubber diaphram 54 across the mouth of a cylinder 55 provided with a respective outlet 56,57. Outlets 56, 57 are connected to a constant volume reciprocating source of hydraulic pressure (such as a reciprocating piston) so that the diaphrams move in unison, one diaphram moving in whilst the other is moving out, such that culture medium in the chambers 50,51 is periodically moved from side to side at a frequency of about 5 Hz.

Disposed in a rectangular section 60 between the two chamber portions is a matrix 61 composed of two bundles of fibres arranged at right angles. The fibres are arranged in alternating planes through the thickness of the matrix. Inlet 62 and outlet 63 are provided for the first bundle arranged in the vertical direction. Inlet 64 and outlet 65 are provided for the second bundle of fibres arranged in the horizontal direction.

An inlet 66 for nutrient solution is provided to the chamber portion 50, which is also provided with a pH sensor 67.

As in the previous two embodiments, a third bundle of fibres may be added for nutrient solution.

To scale-up the capacity of the apparatus, two or more modular rectangular sections may be incorporated as required.

The hollow fibre cell culture apparatus allows the culturing of cells to high concentration, for example up to $10^8$ cells per ml. On a volume basis, this corresponds approximately to a 10% cell concentration. The pH is maintained around 7 by adjustment of the $CO_2$ content of the gaseous stream. The oxygen concentration in the culture medium is arranged to be 10 to 60% of saturation concentration. In order to maintain adequate mixing so as to avoid concentration or pH gradients, the rocking table of the first and second embodiments is arranged to rock at about 1Hz, whilst the pulsating chamber of the third embodiment is arranged to pulsate at about 5Hz. The apparatus is suitable for long culture runs, often of at least 40 days with some cell lines, and is suitable for a wide range of attached and non-attached cells.

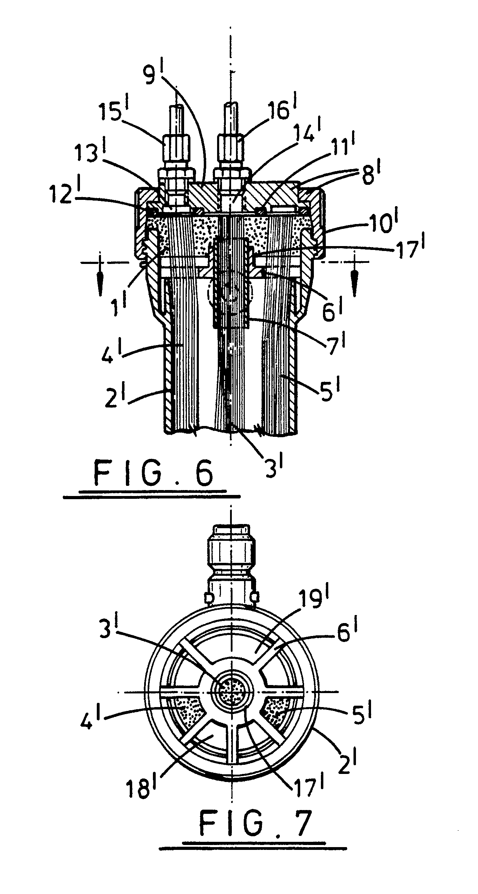

Figures 6 and 7 show a detailed end-cap arrangement for a cylindrical culture chamber having two types of fibre bundle.

In the potting area 1' of the cylindrical housing 2', there is an alternative arrangement of fibre bundles as can be seen from the cross section shown in Figure 7. The two fibre bundles 3' and 4', 5' are separated and fixed by means of a star like plate 6' which has been previously attached to each end of the housing. These plates have holes 18', 19' both in their centers and in their circumferences through which the fibre bundles can be drawn. A capsule 7' made of closely mashed net of a suitable material is wound around the center bundle 3'. Through this it is possible to both center and separate the central fibre bundle 3' from bundle 4', 5' in the outer ring. The sealing of the housing ends, the separation of the fibre bundles in the potting area and the connection of the fibre bundles with the external media circuit are achieved by a screw end cap 8' which consists of two parts. Two circular grooves, for the insertion of O-rings 11', 12', are formed in the bottom 9' of the end cap. These O-rings separate the area of the hydrophilic hollow fibre bundle 3' from that of the hydrophobic bundle 4', 5' in the cutting plane of the potting material and at the same time the whole potting area is sealed off from the outside. In the bottom part depressions 13', 14' are formed between the O-rings 11', 12' and also in the center of the middle O-ring 11'. This enables the formation of two compartments. The connection of these compartments to the corresponding circuit for liquid medium and gaseous medium is achieved by tube connectors 15', 16' which are inserted into the bottom part. The screw part 10' is formed like a swivel nut which presses the bottom part 9' against the potting area when screwed. The potting area is prevented from moving towards the inside of the housing area by means of tube like extensions 17' from the star like plates which partly enter the potting area.

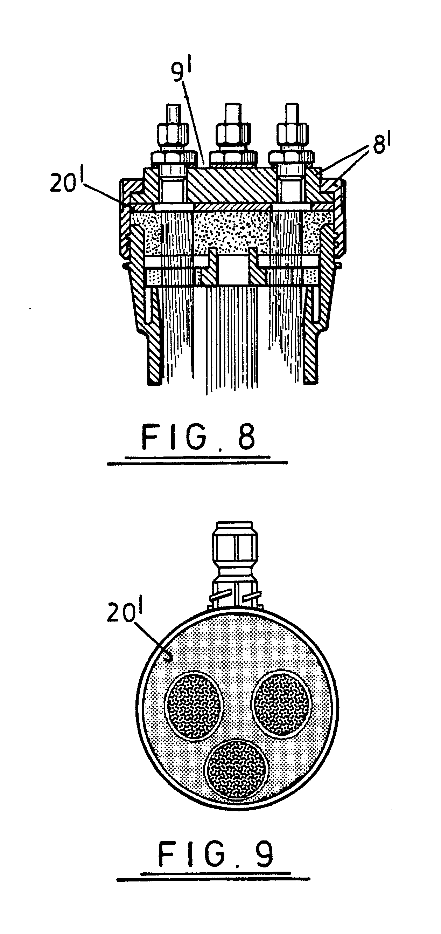

In a further end-cap arrangement shown in Figures 8 and 9 a third or further type <>f fibre bundle can be incorporated. In this arrangement the O-rings 11' and 12' are replaced by a single suitably formed circular gasket 20' with sections corresponding to the exposed ends of the fibre bundles removed. Screwed holes are formed in the end section 9' of the end cap assembly 8' to match the cut away sections of the gasket. By this method three or more separate compartments are formed which connect the respective fibre lumen to the external liquid or gaseous circuits. The connection of these compartments to the external circuits is made by means of screwed tubing connectors. The screw part 10' acting on the end section 9' compresses the gasket 20' against the potting area 1'.

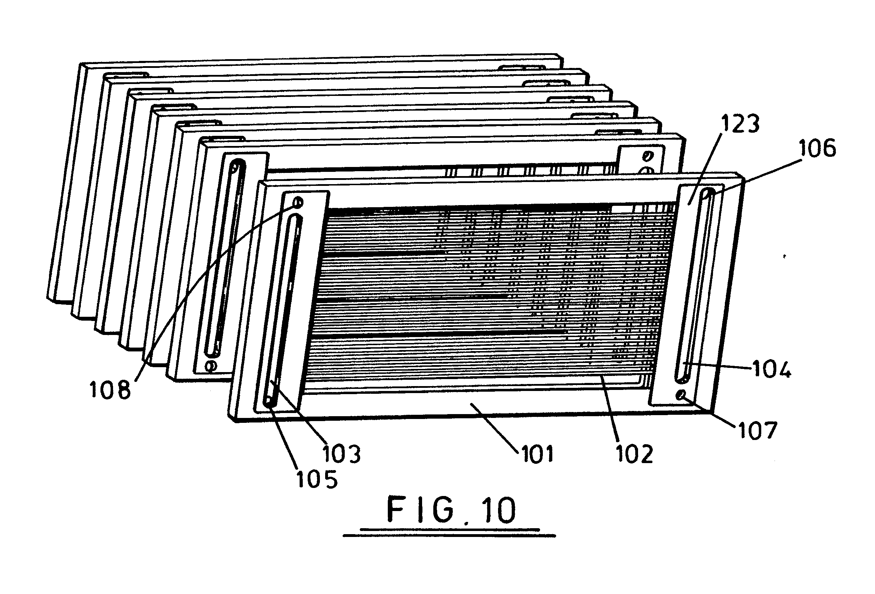

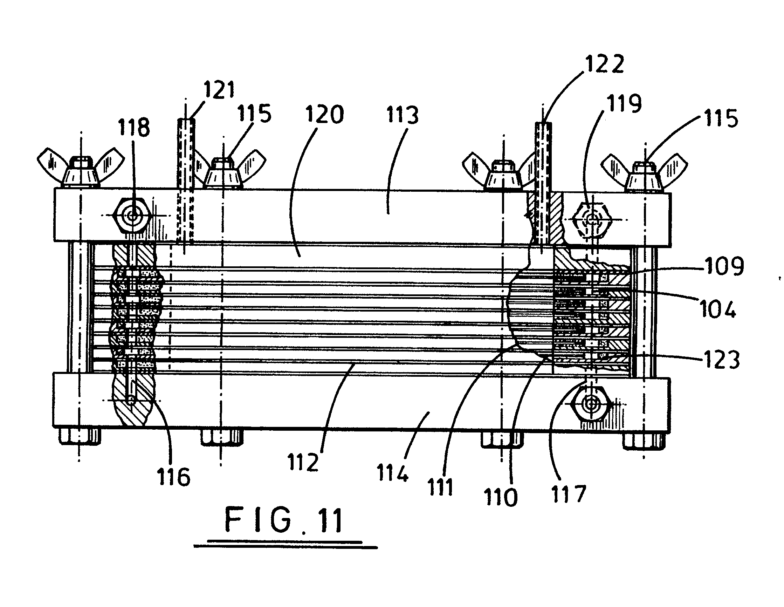

A further modification of the dual fibre culture apparatus is shown in Figures 10 and 11 and consists of a housing made from single frames 101. The hollow fibres 102 are stretched across the frames and are fixed at their ends by a suitable polymeric elastomer potting compound. The fiber openings are accessible by manifold channels 103, 104 which are formed in the potting area 123. By means of preformed holes 105, 106 in the channels, the inflow and outflow of the appropriate media through the channels into every second frame is made possible. A second pair of holes 107, 108 which are separate from both manifold channels form a separate entrance into the second frame provided with the second type of fibres. The frames are separated and sealed from each other by means of suitably formed gaskets 109 shown in Figure 11. The frames are placed together in such a way as to allow the alternative positioning of the hydrophobic 110 and hydrophilic fibres 111 (Figure 11). A reaction chamber 112 is thus formed which is evenly filled with ordered layers of hydrophilic and hydrophibic fibres. The volume of the reaction chamber can be determined by the number of frames. The frames are secured between a top 113 and a bottom plate 114 bolted together by means of nuts and bolts 115 to ensure that the frames are tightly pressed together. Holes 116, 117, 118, 119 in the top and the bottom plate permit entrance and exit of the liquid or gaseous medium to and from the manifold channels in the frames. The frame construction makes it possible to achieve a counter current of the media and a diagonal flow through each frame. In order to achieve a gaseous head space, a spacer frame 120 without hollow fibres, is placed between the top plate and the first hollow fibre frame so that only the hollow fibre frames are immersed in the cell culture medium. The top plate is provided with an inlet 121 for nutrient solution and a sample port 122 in the same way as the device shown in Figure 1.

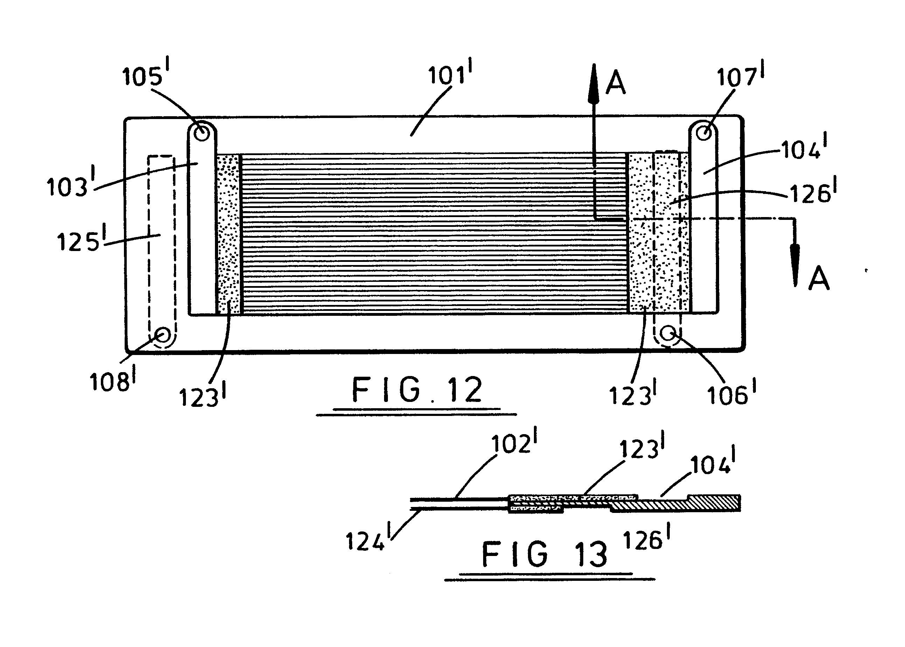

In a further modification of the plate arrangement as shown in Figures 12 and 13, hollow fibres 102' and 124' of two distinct types (for example hydrophilic and hydrophobic) are stretched across a single frame 101'. The ends of the frame are so formed as to provide separate potting areas 123' both on the upper and lower surfaces of the frame. The potting areas are offset to provide appropriate sealing surfaces for the gaskets 109' as shown in Figure 11. Manifold channels 103', 104', 125' and 126' are formed in the potting areas and these connect with holes 105', 106', 107', and 108' respectively. The holes can be so arranged that the plates may be assembled into a group of plates so that the fibres may function in series or in parallel.

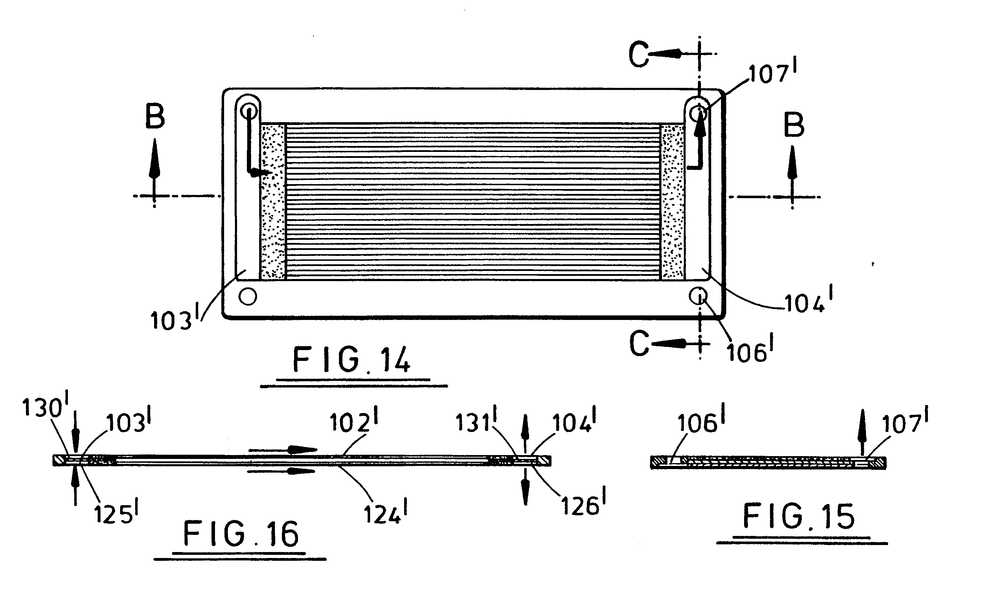

Figures 14 to 16 show a different dual-fibre plate construction which is similar to Figure 12 except that the manifold channels 103', 125' and 104', 126' respectively are arranged to lie above each other; channels 103' and 125' being separated by a web 130' and channels 104' and 126' being separated by a web 31'. Otherwise, the same reference numerals are used as in Figure 12.

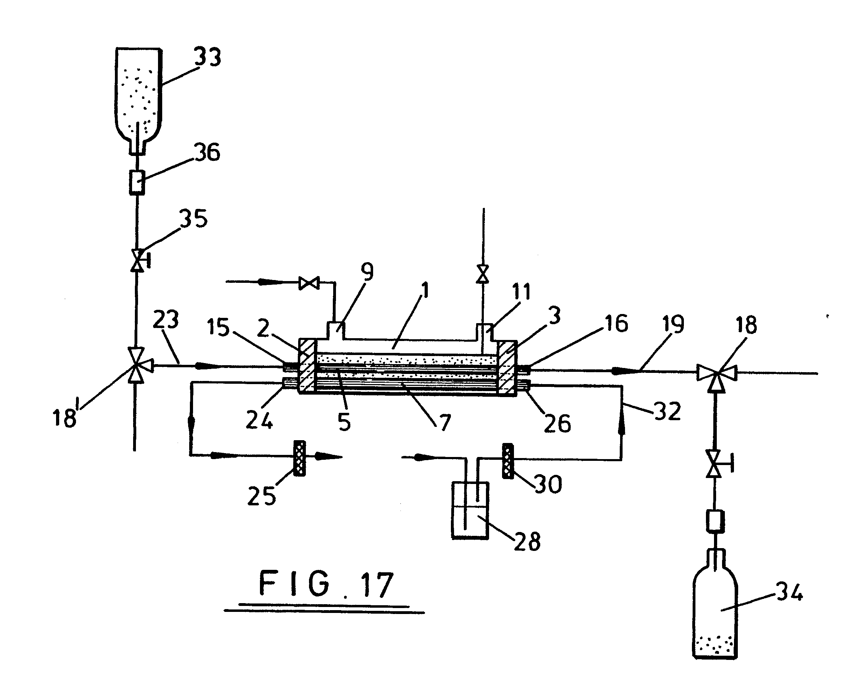

A fifth embodiment of the culture apparatus involves replacement of the medium pump by a hydrostatic pressure driven system, as shown in Figure 17. Analogous parts are shown with the same reference number as in Figure 1. The nutrient solution is filled into a bottle 33 hanging over the reactor chamber 1. The nutrient medium flows through the hydrophilic hollow fibres 5 in a single pass mode and is collected in a further medium collection bottle 34. The flow rate is adjusted by a roller clamp 35 and controlled by a drip chamber 36, similar to those used in medical infusion tube systems. If the composition of the single passed nutrient solution allows the solution to be reused, the nutrient medium can be recycled through the hydrophilic hollow fibres again but in the opposite direction. This is achieved by raising the now filled collection bottle 34 and lowering the empty reservoir bottle 33. This procedure can be repeated until the nutrient composition becomes unsuitable for further use. This arrangement makes it possible to use bottles of presterilized nutrient solutions and to carry out nutrient solution transport without the use of a pump.

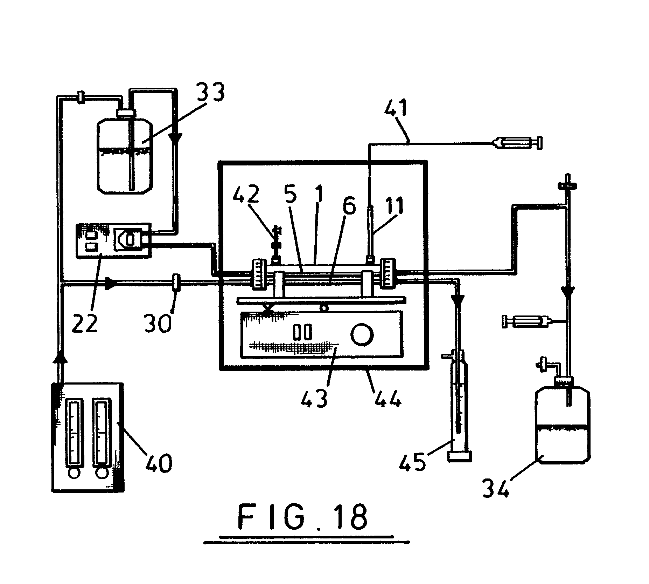

Figure 18 shows a sixth embodiment of the culture apparatus which is broadly similar to the fifth embodiment (and uses analogous reference numerals) but differs in minor details as will become clear from the following Examples carried out in the apparatus.

Example 1

Two hollow fibre bundles 5, 6 were fixed and potted into a polycarbonate dialyser housing 1 (Fresenius AG West Germany) as shown in Figure 18 using a polyurethane elastomer. The two bundles were arranged as two parallel arrays with one bundle 5 sited above the other bundle 6. The lower bundle 6 contained 320 microporous hydrophobic polypropylene hollow fibres with an external diameter of 600 um and wall thickness of 40 um (Enak West Germany). The upper bundle 5 contained 832 hydrophilic polysulfone fibres having an external diamater of 280 um and a wall thickness of 40 um (Fresenius AG PS 600, Nominal molecular weight cutoff 30,000 Daltons). The extra-capillary volume of the culture vessel was approximately 500mls with an internal length of 240 mm and a core of 47 mm. The fibre bundles were connected to external tubing lines and reservoirs as shown in Figure 18. The culture vessel and associated tubing were autoclaved in a standard laboratory autoclave at 121°C for 45 minutes. The culture vessel was connected to the nutrient reservoir and to spent nutrient reservoir in a sterile air work station by means of Luerlok connectors. The reservoirs had been autoclaved to simplify assembly and handling of the equipment. The culture vessel was placed on a variable speed rocking shaker 43 (STR6 Stuart Scientific Ltd), the platform of which was covered by a portable 37°C cabinet 44 (Stuart Scientific). Prior to inoculation the vessel and fibres were thoroughly dried and conditioned by passing dry ambient air containing approximately 7.5% $CO_2$ through the lumen of the polypropylene fibre bundle. The air supply was provided by a small air pump and the $CO_2$ delivered via a pressure regulator from a liquid $CO_2$ bottle 40. The flow of the two streams was controlled by a combination of miniature regulators and "Flotstat" automatic gas flow controllers (Platon Flowbits).

The medium used for the culture within the vessel was RPMI1640 (m404 Northumbria Biologicals Ltd) made up to an initial glucose concentration of 2000 mgs litre $^{-1}$ and supplemented with 10% foetal calf serum (S102 Northumbria Biologicals Ltd.). For the dialysate, RPMI 1640 without serum supplement was used. Phenol Red was incorporated in the medium to give a visual indication of culture pH.

A murine hybridoma cell line (ATTC HB124 Line DB9G8) secreting and IgG to human and porcine insulin was selected for the experiment. A culture in mid growth phase from a tissue culture flask was suspended in fresh medium containing a 10% supplement of foetal calf serum and diluted to an initial cell density of $1.0 \times 10^5$ cells $ml^{-1}$. The culture vessel was inoculated with 250 mls of the culture via the sample port 11 by means of a syringe 41. During this operation the vent 42 to the chamber was opened to permit the displacement of the gas in the head space. After inoculation was completed, the vent and sample lines were closed. The culture filled the reactor to 50% capacity leaving a 250 ml gas head space. The culture was mixed by the rocking action of the platform shaker which was initially set at a rate of 15 cycles per minute with an angular displacement of 6 degrees.

The nutrient stream to the fibre lumen was pumped from the nutrient reservoir 33 placed outside the $37^{oC}$ cabinet by means of a peristaltic pump 22 (101U/R. Watson Marlow) at an initial flow rate of 100 mls $day^{-1}$. The gas supply was controlled to 150 mls $min^{-1}$ at a $CO_2$ concentration of approximately 7.5%. The outlet gas was passed through a sanitising bubble chamber 45. The feed reservoir was top gassed with the air $/CO_2$ mixture to maintain the pH of the medium.

The reactor and the waste stream were sampled daily via the sample lines using a syringe. The cell number in the culture was determined by using a haemocytometer. Cell viability was determined by Trypan blue exclusion staining (T-8154 Sigma Chemical Co.). The concentration of glucose in the culture and the waste stream was assayed by the enzymatic method of Barnham and Trinder (Analyst 1972 Vol92 p142..145). Accumulation of lactate was measured in both samples using the Sigma Lactate kit (Kit 826-UV Sigma Chemical Co.).

The concentration of antibody was determined by a standard "sandwich" ELISA for murine IgG. Plates were coated with antimouse IgG (goat) (M-3014 Sigma Chemical Co.), blocked with BSA and assayed with horseradish peroxidase conjugated antimous IgG (sheep) (SAPU S081-201). TMB (BDH Chemicals) was used as the chromogen and the plates read at 450 nm. The samples were assayed at dilutions of 1:20,000, 1:40,000 and 1:80,000 against a purified IgG standard (I-5381 Sigma Chemical Co.) serially diluted to 16, 8, 4, 2, 1 and 0.5 nanograms per ml.

As the culture proliferated and the rate of glucose consumption increased the flow rate of the dialysate/nutrient stream was adjusted to maintain the residual glucose concentration within the reactor at approximately 1000 mgs $litre^{-1}$.

The observed cell culture density rose to $8.05 \times 10^6$ cells $ml^{-1}$ by day 15 with a viability of 90% before being terminated in day 16. The estimated daily consumption of glucose increased to 3.63 mgs $day^{-1}$ $ml^{-1}$ of culture. The concentration of antibody increased from an initial value of 5.8 ugrams $ml^{-1}$ to a final value on day 16 of 465 ugrams $ml^{-1}$. The outlet waste stream was also assayed for antibody and none was detected. The glucose and lactate concentrations of the reactor and the waster were almost identical at the flow rates employed.

Example 2 (Comparison)

In parallel with Example 1 a comparison experiment was established using a 250 ml Techne stirrer flask inoculated with 250 mls of the same culture at approximately the same initial cell density as the experiment described above using RPMI 1640 with a 10% foetal calf serum supplement. The flask was held in a similar 37°C cabinet mounted on a magnetic stirrer set to a speed of 40rpm. The culture was sampled daily and a maximum cell density of $9.2 \times 10^5$ cells $ml^{-1}$ with a viability of 80% was observed on day 4. After this the viability declined until on day 6 the viability had dropped to less than 2%. The maximum antibody level observed was 42.86 ugrams $ml^{-1}$. This gives a yield of antibody produced of 428.6 ugrams per ml of serum which contrasts with the yield of 4650.0 ugrams per ml of serum for the system of the present invention described in Example 1.

**Claims**

1. A cell culture apparatus which comprises;
- a chamber (1) within which cells may be cultured in a liquid culture medium, the chamber having access port means (9,11) for intrcducing nutrient and cell inoculum and for withdrawing cultured cells and cell-products;
- a first bundle of hollow semipermeable fibres (5) contained within the chamber and having an inlet (15) and an outlet (16) for flowing a stream of aqueous liquid therethrough for removal of cell culture products, in use the products passing through the fibre walls from the cell culture medium into the flowing liquid stream within the fibres; and
- a second bundle of hollow semipermeable fibres (7) within the chamber and having an inlet (26) for an oxygenating gas stream, in use oxygen-containing gas passing through the hollow fibre walls from the gas stream into the culture medium and gaseous waste products passing from the culture medium into the gas stream.

2. An apparatus according to claim 1 which further comprises a third bundle of hollow semipermeable fibres (6) passing through the chamber for flowing nutrient medium and optionally for removal of higher molecular weight metabolites.

3. An apparatus according to claim 1 or 2 wherein the fibres bundles pass through the chamber in a substantially parallel arrangement.

4. An apparatus according to claim 3 wherein the second fibre bundle for flowing oxygen-containing gas is the lowermost fibre bundle.

5. An apparatus according to claim 1 or 2 wherein the culture chamber is at least partially defined by a stack of flat parallel plates (101), each plate comprising a plurality of fibres and the opposite ends of the fibres communicating with respective inlet and outlet manifolds (103,104) provided in the plate; each plate being provided with an inlet hole (105) passing through the plate and communicating with the inlet manifold, and an outlet hole (106) passing through the plate and communicating with the outlet manifold, such that in the stack of plates the inlet holes and outlet holes are in respective alignment to form respective inlet and outlet channels.

6. An apparatus according to claim 5 which comprises two or more series of different plates (101), each series of plates comprising a single type of fibre and forming a respective fibre bundle, and each series being provided with its own inlet and outlet channels.

7. An apparatus according to claim 5 wherein each plate (101) comprises pluralities of at least two types of fibres, each type of fibre forming part of a respective fibre bundle,
the fibres of each type being provided with respective inlet and outlet manifolds communicating with respective inlet and outlet channels.

8. An apparatus according to claim 1 or 2 wherein the fibre bundles are arranged in layers (61) and the fibres of the respective bundles run in substantially equiangularly spaced directions.

9. An apparatus according to any preceding claim wherein the fibres of the first bundle for carrying aqueous liquid comprise a hydrophilic semipermeable membrane material.

10. An apparatus according to any preceding claim wherein the fibres of second bundle for carrying oxygen-containing gas comprise a hydrophobic microporous material.

11. An apparatus according to any preceding claim wherein the ratio of the total fibre surface area to the culture volume is in the range 0.5 to 5.0.

12. An apparatus according to any preceding claim which further comprises mixing means (43) for moving the liquid culture medium relative to the fibre bundles.

13. A method of cultivating cells which comprises
-providing a liquid culture medium containing the cells;
-flowing a stream of aqueous liquid through a first bundle of hollow semipermeable fibres passing through the culture medium for removal of cell culture products from the culture medium into the aqueous liquid;
-flowing an oxygenating gas stream through a second bundle of hollow semipermeable fibres passing through the culture medium for supplying oxygen thereto and for removal of gaseous waste products from the culture medium into the gas stream.

14. A method according to claim 13 wherein the aqueous liquid includes nutrient medium.

15. A method according to claim 13 wherein nutrient is supplied to the culture medium by flowing nutrient medium through a third bundle of hollow semipermeable fibres passing through the culture medium.

16. A method according to claim 15 which comprises removing high molecular weight product from the culture medium via the medium flowing through the third bundle.

F I G . 1

FIG.2

FIG.3

FIG.4

FIG . 5

F I G . 6

F I G . 7

FIG. 8

FIG. 9

F I G . 10

FIG. 11

FIG.12

FIG 13

EP 0 419 234 A2

FIG.14

FIG.15

FIG.16

FIG. 17

EP 0 419 234 A2

FIG.18